# EUROPEAN PATENT SPECIFICATION

(11) **EP 2 515 966 B1**
(45) Date of publication and mention of the grant of the patent: **29.03.2017**
(21) Application number: 10795846.4
(22) Date of filing: 08.12.2010
(51) Int. Cl.: A61M 1/02, A61M 1/36

(54) **METHODS AND APPARATUS FOR COLLECTION OF FILTERED BLOOD COMPONENTS, IN PARTICULAR RED BLOOD CELLS**
VERFAHREN UND VORRICHTUNG ZUR ENTNAHME VON GEFILTERTEN BLUTBESTANDTEILEN, IM BESONDEREN ERYTHROZYTEN
PROCÉDÉS ET DISPOSITIF DE COLLECTE DE COMPOSANTS SANGUINS FILTRÉS, EN PARTICULIER LES GLOBULES ROUGES

(30) Priority: 22.12.2009 US 288994 P
(43) Date of publication of application: 31.10.2012
(73) Proprietor: Terumo BCT, Inc., Lakewood, CO 80215 (US)
(72) Inventor: GIBBS, Bruce, W., Arvada, Colorado 80004 (US)
(74) Representative: Roberts, Mark Peter
(86) International application number: PCT/US2010/059437
(87) International publication number: WO 2011/087631

(56) References cited:
- EP-A1- 1 582 228
- WO-A1-01/17652
- US-A1- 2003 106 861

## Description

The present invention relates generally to the field of extracorporeal blood processing methods and apparatus which are particularly useful in blood component collection, and more particularly, the present invention relates to methods and apparatus for the leukoreduction of red blood cells through a filter and recovery of red blood cells from the filter using a blood component.

One well-known type of extracorporeal blood processing involves an apheresis system and/or procedure in which blood is removed from a donor, directed to a blood component separation device (e.g., centrifuge), and separated into various blood component types (e.g., red blood cells, white blood cells, platelets, plasma). One or more or all of these blood component types may either be collected, and/or treated for therapeutic purposes before storage or return to a patient, while the remainder may simply be returned to the donor or patient. In one such system, only a particular component of interest, such as red cells, is collected with all other blood component types being returned to the donor.

Performance-related factors may affect the commercial viability of an apheresis system. Performance may be judged in terms of the collection efficiency of the apheresis system, which may impact or improve product quality and/or may in turn reduce the amount of processing time and thus decrease operator burden and increase donor convenience. The collection efficiency of a system may of course be gauged in a variety of ways, such as by the amount of a particular blood component type which is collected in relation to the quantity of this blood component type which passes through the apheresis system. Performance may also be evaluated based upon the effect which the apheresis procedure has on the various blood component types. For instance, it is desirable to minimize the adverse effects on the blood component types as a result of the apheresis procedure (e.g., reduce platelet activation).

Another performance-related factor is the end quality of the collected blood component. For example, if red blood cells are the component to be collected, it is generally desirable that such red blood cells be leukoreduced by the removal of white blood cells or leukocytes. White blood cells can present problems to the ultimate recipient of the collected blood component. Transfused products containing white blood cells can provoke immunogenic reactions and viral diseases. Conventionally, filters have been used to remove leukocytes from collected blood products or components. For example, U.S. Patent No. 5,954,971 discloses the use of a filter with an apheresis system for filtering a diluted blood component prior to collection. Other distinctive methods have also been used, and these have generally dictated special preliminary steps such as pre-chilling and/or overnight storage of collected components prior to filtration. Another distinct conventional filtration step is the venting of air at the end of the filtration procedure which has been deemed important for substantial recovery of a remainder portion of the blood component to be processed through a red blood cell filter. Another technique used for leukoreduction is the technique of actively pumping the red blood cells through the leukoreduction filter. Such active pumping, however, may result in cell damage and thus affect the end quality of the collected component.

An apparatus and method for red blood cell filtration in conjunction with apheresis separation is also disclosed in the commonly-owned U.S. Patent 7,052,606. Further background on apheresis red blood cell separation and collection can be found in the PCT publication WO99/11305.

US 2003/0106861 discloses the pre-characterising portion of claim 1.

The invention is defined in the claims.

The present invention generally relates to extracorporeal blood processing. Since each of the various aspects of the present invention may preferably be incorporated into an apheresis system (e.g., whether for blood component collection in which "healthy" cells or other blood components are removed from the donor blood for later transfusion, or for therapeutic "unhealthy" blood component removal), the present invention will be described in preferred relation to such apheresis systems. Apheresis may often imply the return of certain blood components back to the donor. However certain aspects of the present invention may be suited for extracorporeal blood processing applications in which all donated blood components are retained and such are also intended within the scope of the present invention.

An apheresis system which may be used with and/or in one or more aspects of the present invention generally includes at least a blood component separation device (e.g., a membrane-based separation device, and/or a rotatable centrifuge element, such as a rotor and channel combination), which provides the mechanism and/or the forces required to separate blood into its various blood component types (e.g., red blood cells, white blood cells, platelets, and/or plasma). In one preferred embodiment, the separation device includes a centrifuge channel which receives a disposable blood processing vessel. Typically, a donor is fluidly interconnected with the blood processing vessel by an extracorporeal tubing circuit, and preferably the blood processing vessel and extracorporeal tubing circuit collectively define a closed, sterile system. When the fluid interconnection is established, blood may be extracted from the donor and directed to the blood component separation device such that at least one type of blood component may be separated and removed from the blood, either for collection or for therapy.

One aspect of the present invention relates to an extracorporeal blood processing device which is used to provide leukoreduced red blood cells, that in one embodiment includes a disposable assembly which may include one or more flexible tubing lines adjacently interconnected to a blood processing vessel, a collection container interconnected to one of the flexible tubing lines, and a filtration device for filtering a selected separated blood component type such as separated red blood cells. The filtration device is preferably disposed between the blood processing vessel and the collection container. The blood processing device may collect a desired component which will not be returned to the donor and may temporarily collect an undesired component, substantial portions of which will ultimately be returned to the donor. In the present invention, after a desired blood component, such as red blood cells, has been passed through the filtration device, an undesired component, such as buffy coat or plasma, is introduced into an inlet of the filter, and is used to push a residue of the desired component remaining in the filter out of the filter, so that the residue of the desired component can be collected. In connection with this process, the adverbial modifier "after" is intended to mean only post-separation, not requiring the entire overall separation process to be complete.

Prior devices and methods, such as U.S. Patent 7,052,606, have disclosed rinsing or flushing an additive or storage solution through the leukoreduction filter before flowing the red blood cells through the filter, and/or with the red blood cells during filtration thereof and/or after completion of the red blood cell filtration through the leukoreduction filter. Temporary storage of an undesired component and use of that component to rinse or flush residual desired component out of the leukoreduction filter, however, has not been disclosed.

In another aspect, the separated red blood cells may be filtered in a high hematocrit state as they exist after separation in the apheresis system. Here also, filtration may take place during or soon after the overall apheresis process. As above, the phrase "after separation" here does not require completion of the entire separation process. An additive/storage solution may be and preferably is added to the red blood cells before and/or during such filtration. The undesired component (e.g., buffy coat) may then also be flushed through the filter after the red blood cells are filtered therethrough.

The invention may comprise a method for the leukoreduction of red blood cells comprising providing separated red blood cells recently removed from a donor; pushing the separated red blood cells through a leukoreduction filter; collecting the red blood cells in a collection container after pushing the high hematocrit red blood cells through the leukoreduction filter; and diverting a predetermined quantity of another blood component into the leukoreduction filter to flush red blood cells from the filter for collection.

The method may also include providing a blood component path for a blood component other than red blood cells, the blood component path fluidly coupled to an outlet of a blood processing vessel, selectively fluidly coupling the blood component path to a storage location or to a red blood cell path, the blood component path being coupled to the red blood cell path between a red blood cell outlet of the blood processing vessel and the filter, and flowing the predetermined quantity of another blood component through the blood component path into the filter.

Another aspect of the invention may comprise a disposable bag and tubing set for collecting blood components comprising a blood processing vessel adapted to be mounted on a rotor of a centrifugal blood separation apparatus, at least one red blood cell collection bag, a red blood cell path, comprising tubing, the red blood cell path coupling an outlet of the blood processing vessel to said red blood cell collection bag, a filter in the red blood cell path, the filter being interposed between the outlet of the blood cell processing vessel and the red blood cell collection bag, a blood component path for a blood component other than red blood cells, the blood component path fluidly coupled to a second outlet of the blood processing vessel and selectively fluidly coupled to a storage location or to the red blood cell path, the blood component path being coupled to said red blood cell path between the outlet and the filter.

Further features may include an apparatus for separating blood, the apparatus comprising a centrifuge rotor, a blood processing vessel adapted to be mounted on the rotor, at least one red blood cell collection bag, a red blood cell path, comprising tubing, the red blood cell path coupling an outlet of the blood processing vessel to the red blood cell collection bag, a filter in said red blood cell path, the filter being interposed between the outlet of the blood cell processing vessel and the red blood cell collection bag, a blood component path for a blood component other than red blood cells, the blood component path fluidly coupled to a second outlet of the blood processing vessel and selectively fluidly coupled to a storage location or to the red blood cell path, the blood component path being coupled to the red blood cell path between the outlet and the filter.

These and still further aspects of the present invention are more particularly described in the following description of the preferred embodiments presented in conjunction with the attached drawings which are described briefly below.
Fig. 1 is a schematic view of an aphaeresis system.
Fig. 2 illustrates a tubing and bag set including an extracorporeal tubing circuit, a cassette assembly, and collection bag assembly for use with the system of Fig. 1, pursuant to the present invention.
Fig. 3 illustrates a cassette assembly as shown in the set of Fig. 2.
Fig. 4 illustrates a disposable bag assembly for batch processing of units of whole blood.
Fig. 5 is a through section of a centrifuge apparatus and rotor for processing blood in the bag assembly of Fig. 4.
Fig. 6 illustrates the bag assembly of Fig. 4 with certain bags removed from the assembly such that an unwanted blood component could be used to flush red blood cells out of a filter.

The present invention will be described in relation to the accompanying drawings which assist in illustrating the pertinent features hereof. Generally, the primary aspects of the present invention relate to both procedural and structural improvements to a blood apheresis system. However, certain of these improvements may be applicable to other extracorporeal blood processing applications whether any blood components are returned directly to the donor or otherwise; and such are within the scope of the present invention as well.

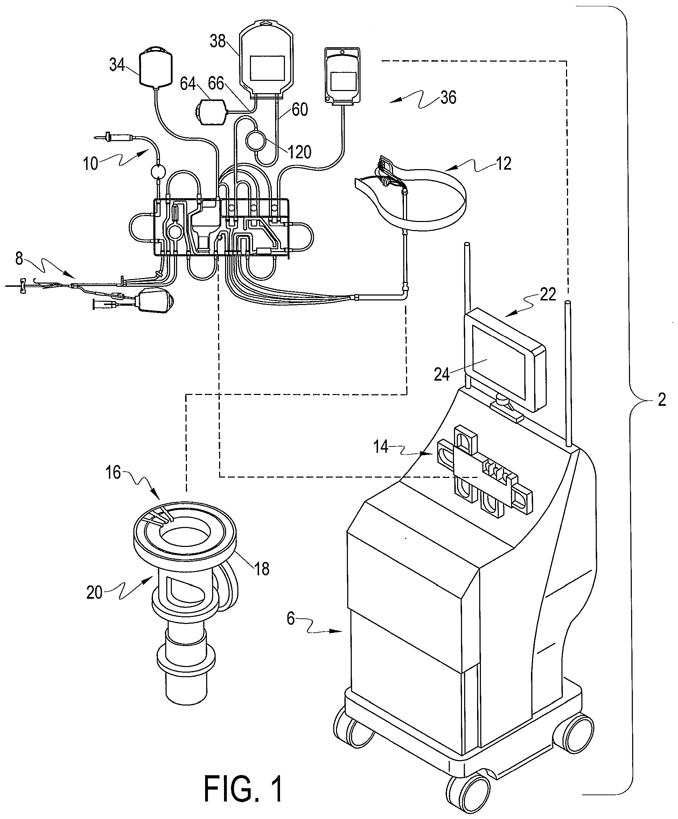

A preferred blood aphaeresis system 2 for use in and/or with the present invention is schematically illustrated in Fig. 1. System 2 preferably provides for a continuous blood component separation process. Generally, whole blood is withdrawn from a donor and is substantially continuously provided to a blood component separation device 6 where the blood is continuously separated into various component types and at least one of these blood component types is preferably continuously collected from the device 6. One or more of the separated blood components may then either be provided for collection and subsequent use by another through transfusion or may be returned to the donor. Therapeutic treatment and near immediate return of certain separated blood components is a viable, yet less common alternative use as well. It is also understood that for therapeutic treatment the blood may be separated into components with filtration using the principles of the instant invention and as described below at a patient's bedside for return to such patient.

In the blood aphaeresis system 2, blood is withdrawn from the donor and directed through a pre-connected bag and tubing set 8 which includes an extracorporeal tubing circuit 10 and a blood processing vessel 12 which together define a closed, sterile and disposable system. The set 8 is preferably disposable and is adapted to be mounted on and/or in the blood component separation device 6. The separation device 6 preferably includes a pump/valve/sensor assembly 14 for interfacing with the extracorporeal tubing circuit 10, and a channel assembly 16 for interfacing with the disposable blood processing vessel 12.

The channel assembly 16 may include a channel housing 18 that is rotatably interconnected with a centrifuge rotor assembly 20, which provides the forces required to separate blood into its various blood component types by centrifugation. The blood processing vessel 12 may be fitted within the channel housing 18. When connected as described, blood can be flowed substantially continuously from the donor, through the extracorporeal tubing circuit 10, and into the rotating blood processing vessel 12. The blood within the blood processing vessel 12 may then be continuously separated into various blood component types and at least one of these blood component types (platelets, plasma, or red blood cells) is preferably continually removed from the blood processing vessel 12. Blood components which are not being retained for collection or for therapeutic treatment are preferably also removed from the blood processing vessel 12 and returned to the donor via the extracorporeal tubing circuit 10. Various alternative aphaeresis systems (not shown) may also make use of the present invention, including batch processing systems (non-continuous inflow of whole blood or non-continuous outflow of separated blood components) or smaller scale batch or continuous RBC/plasma separation systems, whether or even if no blood components may be returned to the donor.

Operation of the blood component separation device 6 is preferably controlled by one or more processors included therein, and may advantageously comprise a plurality of embedded computer processors to accommodate interface with ever-increasing PC user facilities (e.g., CD ROM, modem, audio, networking and other capabilities). In order to assist the operator of the aphaeresis system 2 with various aspects of its operation, the blood component separation device 6 preferably includes a graphical interface 22 with an interactive touch screen 24.

Further details concerning the operation of a preferred aphaeresis system, such as the Terumo Trima® System and the Trima® Accel^{™} System (available from the assignee of this application, Terumo BCT, Inc., Lakewood, Colorado) may be found in a plurality of publications, including, for example, WO99/11305 and U.S. Patents No. 5,653,887; No. 5,676,644; No. 5,702,357; No. 5,720,716; No. 5,722,946; No. 5,738,644; No. 5,750,025; No. 5,795,317; No. 5,837,150; No. 5,919,154; No. 5,921,950; No. 5,941,842; and No. 6,129,656; among numerous others. A plurality of other known aphaeresis systems may also be useful herewith, as for example, the Baxter CS3000®, Amicus®, Autopheresis-C®, and Alyx systems or the Haemonetics MCS® and MCS®+, or the Fresenius COM.TEC^{™} and AS-104^{™} or like systems.

### Disposable Set: Extracorporeal Tubing Circuit

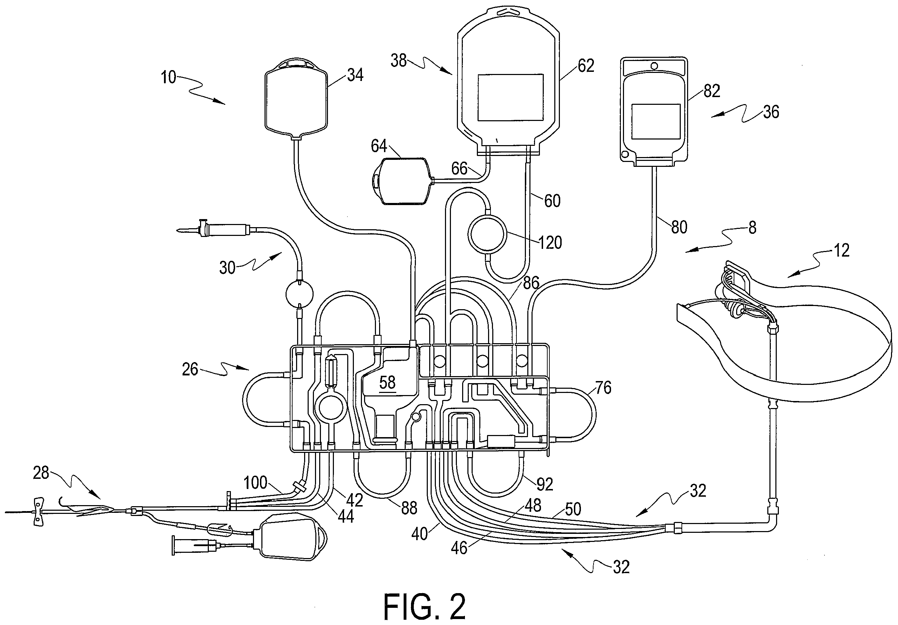

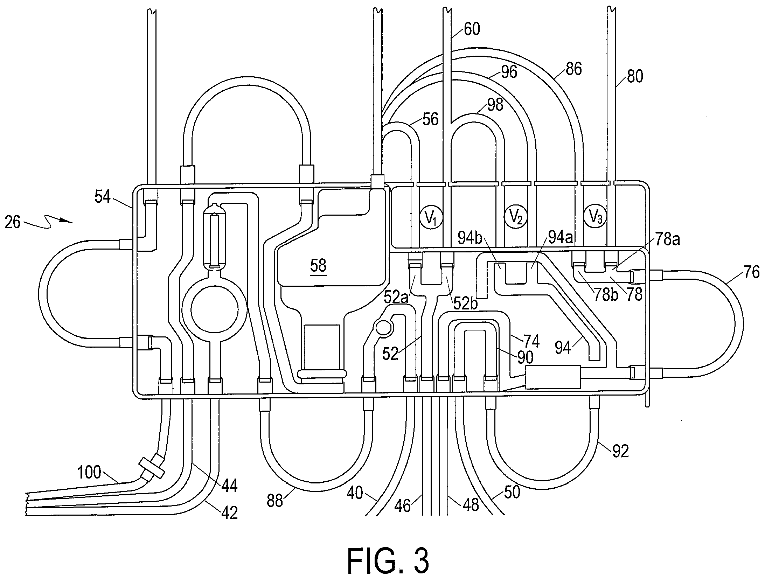

As illustrated in Figs. 2 and 3, the pre-connected extracorporeal tubing circuit 10 is shown which may include a cassette assembly 26 and a number of tubing/collection assemblies 28, 30, 32, 34, 36, and 38 interconnected therewith. Preferably, a blood removal/return tubing assembly 28 provides a single needle interface between a donor and the remainder of the tubing circuit 10 (although a two-needle set-up may also be used). At least two lines 42, 44 are provided in assembly 28 (see Fig. 3) for removal of blood from and return of components to the donor. This embodiment includes a cassette assembly 26 interconnected between the tubing assembly 28, which connects the donor thereto, and blood inlet/blood component outlet tubing line sub-assembly 32, which provides the interface between cassette assembly 26 and blood processing vessel 12. Line 40 transports blood to the processing vessel 12. Three lines 46, 48 and 50 are shown in Figs. 2 and 3 for transport of blood and components from the processing vessel 12. An anticoagulant tubing assembly 30, a plasma collection tubing and bag assembly 36, a red blood cell collection assembly 38, and a vent bag tubing line sub-assembly 34 are also interconnected with cassette assembly 26 in this embodiment. The extracorporeal tubing circuit 10 and blood processing vessel 12 are preferably pre-interconnected to yield a closed, pre-sterilized disposable assembly for a single use.

Emanating from vessel 12 is an RBC outlet tubing line 46 of the blood inlet/blood component tubing assembly 32 which is interconnected with integral RBC passageway 52 of cassette 54 of cassette assembly 26 (see Figs. 2 and 3). The integral RBC passageway 52 includes first spur 52a and second spur 52b. The first spur 52a is interconnected with RBC return tubing loop 56 to return separated RBCs to a donor. For such purpose, the RBC return tubing loop 56 is preferably interconnected to the top of a blood return reservoir 58 of the cassette assembly 26. The second spur 52b may, as preferred herein, be connected with an RBC collection tubing assembly 38 (see Figs. 2 and 3, for example) for collecting RBCs during use. RBC collection tubing and bag assembly 38 preferably includes RBC collector tubing line 60 which communicates with spur 52b, a filter 120, an RBC collection reservoir or bag 62, and an air removal bag 64. The air removal bag 64 is attached to the RBC collection bag 62 by a tubing line 66 which may have an optional clamp attached thereto. The RBC collection tubing line and container sub-assembly 38 is preferably a pre-connected part of the disposable assembly 8.

In a portion of the cassette assembly 26, plasma tubing 48 of blood inlet/blood component tubing assembly 32 (see Figs. 2 and 3) interconnects with a first integral plasma passageway 74 (see Fig. 3) of cassette assembly 26 (note, this is preferably a plasma collection sub-system; however, other components such as platelets could alternatively be collected here or with a similar arrangement). Cassette assembly 26 further includes a pump-engaging, plasma tubing loop 76 interconnecting the first integral plasma passageway 74 and a second integral plasma passageway 78. The second integral plasma passageway 78 includes first and second spurs 78a and 78b. The first spur 78a is interconnected to the plasma collection tubing assembly 36 via tubing line 80. The plasma collection tubing assembly 36 may be employed to collect plasma during use and includes plasma collector tubing 80 and plasma collection bag 82. A slide clamp may be provided on plasma collector tubing 80. The second spur 78b of the second integral plasma passageway 78 is interconnected to a plasma return tubing loop 86 to return plasma to donor/patient. For such purpose, the plasma return tubing loop 86 is interconnected with the blood return reservoir 58. A valve V3 selectively opens line 80 and closes loop 86 or conversely closes line 80 and opens loop 86.

A buffy coat extraction line 50 carries buffy coat, comprising white blood cells, platelets and plasma, from the separation chamber 12 through an integral passageway 90 to a pump engaging loop 92. The pump loop 92 connects to an integral passage 94 having a first spur 94a and a second spur 94b. The first spur 94a is connected to a return loop 96 that allows unwanted buffy coat to be returned to the donor through the reservoir 58. The second spur 94b communicates with an interconnect loop 98 that is joined to the red blood cell collection line 60. After red blood cells have been collected in the red blood cell bag 62, the red blood cell line may be closed by a valve V1 located between the junction between line 60 and the RBC return tubing loop 56. Valve V2 is rotated to close the return loop 96 and open the interconnect loop 98, thereby diverting an unwanted blood component, e.g., buffy coat, into the red blood cell collect line 60. Operation of a peristaltic pump engaging pump loop 92 allows insertion of a pre-determined quantity of buffy coat into the collect line 60. The buffy coat can therefore push red blood cells out of the filter 120 and into the collect bag 62 without diluting the collected red blood cells.

Most portions of the tubing assemblies 28, 30, 32, 36, 34, and 38 and cassette assembly 26 are preferably made from plastic components including, for example, polyvinyl chloride (PVC) tubing lines, that may permit visual observation and monitoring of blood/blood components during use. It should be noted that thin-walled PVC tubing may be employed for approved, sterile docking (i.e., the direct connection of two pieces of tubing line) for the RBC collector tubing lines 60, inter alia. All tubing lines are pre-connected before sterilization of the total disposable assembly to assure that maximum sterility of the system is maintained. A highly desirable advantage of pre-connection of all of the elements of the tubing circuit including the collection bag sub-assembly 38 involves the complete pre-assembly and then sterilization hereof after pre-assembly such that no sterile docking is later necessary (spike addition of storage solution excepted). Thus, the costs and risks of sterile docking are eliminated. Alternatively, thicker-walled PVC tubing may be employed for approved, sterile docking RBC collector tubing lines 60, inter alia.

As mentioned, a cassette assembly 26 in the embodiment of Fig. 3, may be mounted upon and operatively interface with the pump/valve/sensor assembly 14 of a blood component separation device 6 during use. Further details of an aphaeresis system set-up including the loading and interaction of a disposable assembly 8 with a blood component separation device 6, may be found in the above-listed patents, inter alia, and are not exhaustively repeated here.

### Operation of Extracorporeal Tubing Circuit and Blood Component Separation Device

Priming and various other operations of the aphaeresis process are preferably carried out as set forth in the above-listed patents. During a blood removal, whole blood will be passed from a donor into tubing line 42 of blood removal/return tubing assembly 28 and is then transferred to blood component separation device 6. Anti-coagulant (not shown) may be added through anti-coagulant tubing assembly 30 in a controlled fashion by action of a pump on a pump engaging loop leading to anti-coagulant line 100. At device 6, the blood is pumped via loop 88 (see Fig. 3) to the processing vessel 12 via the cassette assembly 26 and line 40 of the blood inlet/blood component tubing assembly 32 (Figs. 2 and 3). Separation processing then occurs on a substantially continuous basis in vessel 12; i.e., blood flows therein, is separated and flows as separated components therefrom. After separation processing in vessel 12 (though separation is continuously occurring), uncollected blood components are transferred from the processing vessel 12 to and through cassette assembly 26, into and reservoir 58 (Figs. 2 and 3) of cassette 26 up to a predetermined level at which the blood component separation device 6, in a single needle operation, may (though in a continuous system, need not) pause the blood removal submode and initiate a blood return submode wherein these uncollected and/or treated components may be returned to the donor. As such, these accumulated components may be pumped through a pump engaging loop into the blood return tubing line 44 of blood removal/return tubing assembly 28 and back into the donor. During the single needle blood return mode, when the accumulated return blood components in reservoir 58 are removed down to a predetermined level, blood component separation device 6 will then automatically end the blood return submode. This preferably will also automatically serve to reinitiate or continue the blood removal submode. The cycle between blood removal and blood return submodes will then continue until a predetermined amount of collected blood components have been harvested. In an alternative dual needle scheme, as is known in the art, blood may be continually removed from and blood components continually returned to a donor. The detailed mechanisms for such operations, including controlling the pumps, for example, are not shown or described in detail herein.

Also, certain components may be collected simultaneously or consecutively one after the other. In one example, plasma may be collected concurrently with collection of RBCs. In the primary example shown in Figs. 1-3, two components are shown being collected, RBCs in the RBC sub-assembly 38 and plasma in assembly 36. When a sufficient quantity of one or the other is collected, further separated portions of such a component are returned to the donor with any other uncollected components, until a sufficient quantity of all components are collected. One or two selected components may be collected with all other components being returned to the donor.

With specific reference to Figs. 2 and 3, in normal operation, whole blood will pass from the donor through the needle and blood removal tubing assembly 28, cassette assembly 26 and blood inlet tubing line 40 to processing vessel 12. The whole blood will then be separated in vessel 12. Also, a platelet stream or a plasma (buffy coat) stream may be separated herein and be either collected in a collector assembly 36, or diverted to reservoir 58 for ultimate return to the donor. Separated plasma may be flowed from the processing vessel 12 by way of line 48 through cassette 26 via loop 76 and line 80 for collection in the container 82 for plasma or diverted to reservoir 58 through spur 78b, and line 86. After red blood cells have been collected in the red blood cell bag 62, the red blood cell line may be closed by a valve V1 located between the junction between line 60 and the interconnect loop 98. Valve V2 is rotated to close the return loop 96 and open the interconnect loop 98, thereby diverting an unwanted blood component, e.g., buffy coat, into the red blood cell collect line 60. Operation of a peristaltic pump engaging pump loop 92 allows insertion of a pre-determined quantity of buffy coat into the collect line 60. The buffy coat can therefore push red blood cells out of the filter 120 and into the collect bag 62 without diluting the collected red blood cells.

### Aphaeresis Protocol

One preferred protocol, which may be followed for performing an aphaeresis procedure relative to a donor utilizing the described system 2, will now be summarized. Initially, an operator loads the disposable plastic assembly 8 in and/or onto the blood component separation device 6. According hereto, the operator hangs the various bags on hooks on the blood component separation device 6. If one is used, the operator then also loads the cassette assembly 26 on the device 6 and/or the blood processing vessel 12 within the channel housing 18 as mounted on the centrifuge rotor assembly 20 in the machine 6.

With the extracorporeal tubing circuit 10 and the blood processing vessel 12 loaded in the described manner, the donor may then be fluidly interconnected with the extracorporeal tubing circuit 10 by inserting an access needle of the needle/tubing assembly 28 into the donor. In addition, the anticoagulant tubing assembly 30 (see Fig. 2) is primed and the blood removal/return tubing assembly 28 is primed preferably with blood from the donor. The blood processing vessel 12 is also primed for the aphaeresis procedure. In one embodiment, a blood prime may be used in that blood will be the first liquid introduced into the blood processing vessel 12. During the priming procedure, as well as throughout the remainder of the aphaeresis procedure, blood may be flowed into the vessel 12, blood components are separated from each other and one or more components is removed from the blood processing vessel 12.

The preferred blood aphaeresis system 2 provides for contemporaneous separation of a plurality of blood components during blood processing, including the separation of red blood cells (RBCs) and plasma. In turn, such separated blood components may be selectively collected in corresponding storage reservoirs or immediately or after a minor delay returned to the donor during respective blood return submodes (or substantially constantly in a two-needle setup). In one approach where more than one blood component is to be collected, such as both plasma (and/or platelets) and RBCs, blood aphaeresis system 2 may be used to collect plasma (and/or if desired separated platelets), during a time period(s) separate from the collection of red blood cells. These components may also be collected simultaneously.

To initiate the RBC collection phase, blood component separation device 6 provides an appropriate control signal to the RBC divert valve assembly V1 so as to direct the outflow of the separated RBCs removed from blood processing vessel 12 via line 46 into the RBC collection system 38 through tubing line 60 and filter 120 into collection container 62. The separated RBCs are preferably not pumped out of vessel 12 for collection, but instead are flowed out vessel 12 and through extracorporeal tubing circuit 10 by the pressure of the blood inlet flow to vessel 12. The inlet blood is pumped into vessel 12 via loop 88 of cassette 26. Trauma to the collected RBCs would thereby be minimized.

Following the separation and collection of the desired quantity of red blood cells, blood separation device 6 may then provide a control signal to the RBC divert assembly so as to divert any further RBC flow back to the donor via loop 56, reservoir 58 and return line 44. After red blood cells have been collected in the red blood cell bag 62, the red blood cell line may be closed by a valve V1 located between the junction between line 60 and the RBC return tubing loop 56. Valve V2 is rotated to close the return loop 96 and open the interconnect loop 98, thereby diverting an unwanted blood component, e.g., buffy coat, into the red blood cell collect line 60. Operation of a peristaltic pump engaging pump loop 92 allows insertion of a pre-determined quantity of buffy coat into the collect line 60. The buffy coat can therefore push red blood cells out of the filter 120 and into the collect bag 62 without diluting the collected red blood cells. Additionally, if further blood processing is not desired, rinse back procedures may be completed.

Any air from bag 62, or air caught between the incoming RBCs and bag 62 is ultimately removed to air removal bag 64 through tubing line connection 66. The air is evacuated to air removal bag 64 prior to the flow of the incoming RBCs or is evacuated by the flow of the incoming RBCs. Air can also be vented prior to the separation process by initially running the return pump, (not shown) of the aphaeresis system. Removal of air may also be achieved by other known (though less desirable here) methods, including, for example, hydrophobic vents and/or by-pass lines.

Reservoir 58 is coupled to level and photo sensors, heretofore used to detect presence and quantity of separated RBC blood component. In addition, if plasma is pumped into the reservoir 58, the presence and quantity of the plasma in the reservoir can be detected by the separation device 6 through the same sensors. The plasma can be pumped out of the reservoir 58 by a peristaltic pump engaging a return tubing loop.

Upon completion of chasing with buffy coat, the collection bag 62 may be separated from the rest of the set 8. The separation may be made by a clamp or by RF sealing of the tubing line 60 and then separating in accordance with U.S. Patent Nos. 5,345,070 and 5,520,218, inter alia, along the RF-sealed portion of the tubing line. Other well known methods can also be used to close the tubing line and then also separate the RBC collection system 38 from the remainder of the disposable assembly 8.

Another embodiment of the method of the present invention may be implemented with apparatus such as the Orbisac (TM) blood processing system or the Atreus (TM) blood processing system, both available from Terumo BCT, Inc., the assignee of this application, or with apparatus for simultaneously processing multiple units of collected whole blood, such as the apparatus described in US Provisional Application 61/267,484. Implementation of the method will be described in connection with the apparatus of US Provisional Application 61/267,484.

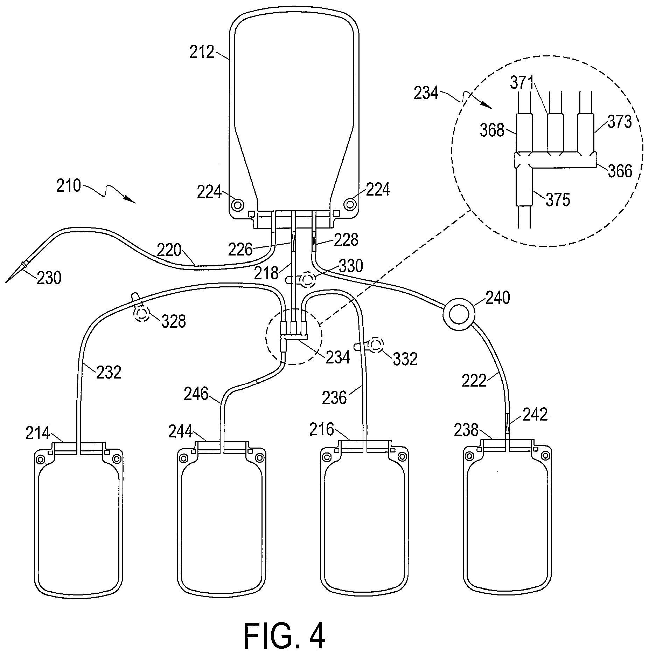

Figure 4 shows an example of a set 210 of bags adapted to be used for the separation of a composite liquid (e.g. whole blood) into at least one component (e.g. plasma, platelets, or both) and a second component (e.g. red blood cells). This bag set comprises a flexible primary separation bag 212 and two flexible component bags 214, 216 connected thereto.

When the composite liquid is whole blood, the separation bag 212 has two purposes, and is successively used as a collection bag and as a separation bag. It is intended to initially receive a discrete volume of whole blood from a donor (usually about 500 ml) and to be used later as a separation chamber in a separation apparatus. The separation bag 212 is flat and generally rectangular. It is made of two sheets of plastic material that are welded together so as to define there between an interior space having a main rectangular portion connected to a triangular proximal portion. A first tube 218 is connected to a proximal end of the triangular portion, and a second tube 220 and a third tube 222 are connected on opposite sides adjacent the first tube 218. The proximal ends of the three tubes 218, 220, 222 are embedded between the two sheets of plastic material so as to be parallel. The separation bag 212 further comprises a hole 224 in each of its two proximal corners that are adjacent to the three tubes 218, 220, 222. The holes 224 may be used to secure the separation bag to a separation cell on a centrifugal blood separation apparatus.

The separation bag initially contains a volume of anti-coagulant solution (typically about 63 ml of a solution of citrate phosphate dextrose for a blood donation of about 450 ml). The first and third tubes 218, 222 are fitted at their proximal ends with a breakable stopper 226, 228 respectively, blocking liquid flow therethrough. The breakable stopper is sometimes called a "frangible". The second tube 220 is a collection tube having a needle 230 connected to its distal end. At the beginning of a blood donation, the needle 230 is inserted in the vein of a donor and blood flows into the separation bag 212. After a desired volume of blood has been collected in the separation bag 212, the collection tube 220 is sealed and cut, disconnecting the needle from the bag set 210. Alternatively, previously collected blood may be transferred to the separation bag 212 through the collection tube 220, with or without the use of the needle 230.

The first component bag 214 is intended for receiving a plasma component. The bag 214 is flat and substantially rectangular. It is connected through a plasma collection tube 232 and an asymmetric manifold 234 to the first tube 218. The second component bag 216 is intended for receiving a platelet component. The second component bag 216 is also flat and substantially rectangular. It is connected through a platelet collection tube 236 and the asymmetric manifold 234 to the first tube 218. A third component bag 238 is intended to receive a red blood cell component (which may be washed), from the primary bag 212. Red blood cells may be drained through tube 222, which may include a filter 240, into third component bag 238. A breakable stopper 242 or frangible in tube 222 prevents premature flow of red blood cells into the third component bag 238.

A waste product bag 244 may be provided to receive white blood cells. This blood component may also be called "buffy coat", "leukopack", or a "minus product" and is denser than either platelets or plasma, but less dense than red blood cells. The white blood cells pass through the asymmetrical manifold 234 and a waste product tube 246 and are temporarily stored in the waste product bag 244.

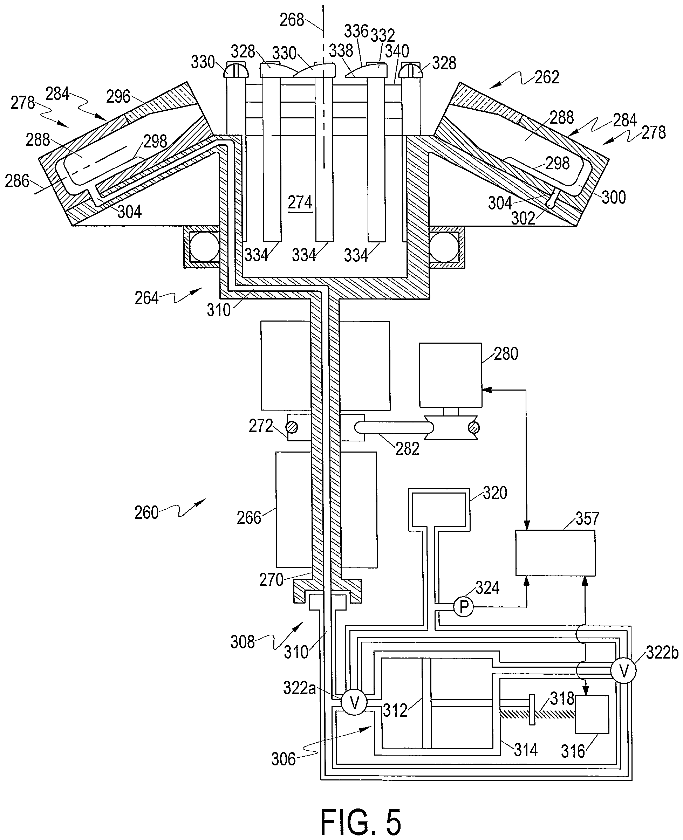

Figure 5 shows an embodiment of an apparatus 260 for simultaneously separating by centrifugation four discrete volumes of a composite liquid. The apparatus comprises a centrifuge 262 adapted to receive four of the sets 210 of bags shown in Figure 4, with the four discrete volumes of a composite liquid contained in the four primary separation bags 212; a component transferring means for transferring at least one separated component from each separation bag into a component bag connected thereto. The apparatus 260 may further comprise means for washing a residual high hematocrit red blood cell component.

The centrifuge 262 comprises a rotor 264 that is supported by a bearing assembly 267 allowing the rotor 264 to rotate around a rotation axis 268. The rotor comprises a cylindrical rotor shaft 270 to which a pulley 272 is connected; a storage means comprising a central cylindrical container 274 for containing component bags, which is connected to the rotor shaft 270 at the upper end thereof so that the longitudinal axis of the rotor shaft 270 and the longitudinal axis of the container 274 coincide with the rotation axis 268. Four identical separation cells 278 are coupled to the central container 274 so as to form a symmetrical arrangement with respect to the rotation axis 268. The centrifuge further comprises a motor 280 coupled to the rotor by a belt 282 engaged in a groove of the pulley 272 so as to rotate the rotor about the rotation axis 268.

Each separation cell 278 comprises a container 284 having the general shape of a rectangular parallelepiped. The separation cells 278 are mounted on the central container 274 so that their respective median longitudinal axes 286 intersect the rotation axis 268, so that they are located substantially at the same distance from the rotation axis 268, and so that the angles between their median longitudinal axes 286 are substantially the same (i.e. 90 degrees). The median axes 286 of the separation cells 278 are inclined downwardly with respect to a plane perpendicular to the rotation axis 268.

Each container 284 comprises a cavity 288 that is so shaped and dimensioned as to loosely accommodate a separation bag 212 full of liquid, of the type shown in Figure 3. The cavity 288 (which will be referred to later also as the "separation compartment") is defined by a bottom wall, which is the farthest to the rotation axis 268, a lower wall that is the closest to the container 274, an upper wall opposite to the lower wall, and two lateral walls. The cavity 288 comprises a main part, extending from the bottom wall, which has substantially the shape of a rectangular parallelepiped with rounded corners and edges, and an upper, or proximal, part, which has substantially the shape of a prism having convergent triangular bases. In other words, the upper part of the cavity 288 is defined by two sets of two opposing walls converging towards the central median axis 286 of the container 284. One interesting feature of this design is that it causes a radial dilatation of a thin layer of a minor component of a composite fluid (e.g. the platelets in whole blood) after separation by centrifugation, and makes the layer more easily detectable in the upper part of a separation bag. This also reduces mixing between component layers by providing a gradual, funnel-like transition into the tube. The two couples of opposite walls of the upper part of the separation cell 278 converge towards three cylindrical parallel channels (not shown), opening at the top of the container 284, and through which, when a separation bag 212 is set in the container 284, the three tubes 218, 220, 222 extend.

The container 284 also comprises a hinged lateral lid 296, which is comprised of an upper portion of the external wall of the container 284. The lid 296 is so dimensioned as to allow, when open, an easy loading of a separation bag 212 full of liquid into the separation cell 278. The container 284 comprises a locking means (not shown) by which the lid 296 can be locked to the remaining part of the container 284. The container 284 also comprises a securing or locating means for securing or locating a separation bag 212 within the separation cell 278. The bag securing or locating means comprises two pins (not shown) protruding on the internal surface of the lid 296, close to the top of separation cell 278, and two corresponding recesses in the upper part of the container 284. The two pins are so spaced apart and dimensioned as to fit into the two holes 224 in the upper corners of a separation bag 212.

The separation apparatus further comprises a component transferring means for transferring at least one separated component from each separation bag into a component bag connected thereto. The component transferring means comprises a squeezing system for squeezing the separation bags 212 within the separation compartments 288 and causing the transfer of separated components into component bags 214, 216. The squeezing system comprises a flexible diaphragm 298 that is secured to each container 284 so as to define an expandable chamber 300 in the cavity thereof. More specifically, the diaphragm 298 is dimensioned so as to line the bottom wall of the cavity 288 and a large portion of the lower wall of the cavity 288. The squeezing system further comprises a peripheral circular manifold 302 that forms a ring. Each expansion chamber 300 is connected to the manifold 302 by a supply channel 304 that extends through the wall of the respective container 284, close to the bottom thereof. The squeezing system further comprises a hydraulic pumping station 306 for pumping a hydraulic liquid in and out the expandable chambers 300 within the separation cells 278. The hydraulic liquid is selected so as to have a density slightly higher than the density of the densest of the components in the composite liquid to be separated (e.g. the red blood cells, when the composite liquid is blood). As a result, during centrifugation, the hydraulic liquid within the expandable chambers 300, whatever the volume thereof, will generally remain in the most external part of the separation cells 278. The pumping station 306 is connected to the expandable chambers 300, through a rotary seal 308, by a duct 310 that extends through the rotor shaft 270, through the bottom and lateral wall of the central container 274, and, radially outwardly where it connects to the manifold 302. The pumping station 306 comprises a piston pump having a piston 312 movable in a hydraulic cylinder 314 fluidly connected via the rotary seal or fluid coupling 308 to the rotor duct 310. The piston 312 is actuated by a brushless DC motor 316 that moves a lead screw 318 linked to a piston rod. The hydraulic cylinder 314 is also connected to a hydraulic liquid reservoir 320 having an access controlled by two valves 322a, 322b for selectively allowing the introduction or the withdrawal of hydraulic liquid into and from a reciprocating hydraulic circuit including the hydraulic cylinder 314, the rotor duct 310 and the expandable hydraulic chambers 300. A pressure gauge 324 is connected to the hydraulic circuit for measuring the hydraulic pressure therein.

The separation apparatus further comprises four sets of three pinch valves 328, 330, 332 that are mounted on the rotor around the opening of the central container 274. Each set of pinch valves 328, 330, 332 faces one separation cell 278, with which it is associated. The pinch valves 328, 330, 332 are designed for selectively blocking or allowing a flow of liquid through a flexible plastic tube, and selectively sealing and cutting a plastic tube. Each pinch valve 328, 330, 332 comprises an elongated cylindrical body 334 and a head 336 having a jaw 338 forming a gap that is defined by a stationary lower plate or anvil 340 and the jaw 338 movable between a "load" position, an "open" position, and a "closed" position. The gap is so dimensioned that one of the tubes 218, 222, 236, 246 of the bag sets shown in Figure 4 can be snuggly engaged therein when the jaw is in the open position. The elongated body contains a mechanism for moving the jaw and it is connected to a radio frequency generator that supplies the energy necessary for sealing and cutting a plastic tube. The pinch valves 328, 330, 332 are mounted inside the central container 274, adjacent the interior surface thereof, so that their longitudinal axes are parallel to the rotation axis 268 and their heads protrude above the rim of the container 274. The position of a set of pinch valves 328, 330, 332 with respect to a separation bag 212 and the tubes 232, 236, 246 connected thereto when the separation bag 212 rests in the separation cell 278 associated with this set of pinch valves 328, 330, 332 is shown in dotted lines in Figure 4. Electric power is supplied to the pinch valves 328, 330, 332 through a slip ring array 266 that is mounted around a lower portion of the rotor shaft 270.

Loading a multi-unit blood separator with a plurality of bag sets 210 can be time-consuming and repetitive. Rapid placement of tubes, such as tubes 218, 232, 236 and 246, is enhanced by the ability of the valve jaws in the "load" position to swing completely clear of a track or groove adapted to receive a tube. Accurate placement of the tubes is enhanced by the use of the asymmetrical manifold 234. The manifold is comprised of relatively rigid plastic and forms a junction for at least three, preferably four, flexible tubes. Connections for the tubes are asymmetrically spaced around the manifold. As shown in Figure 4, an embodiment of the asymmetrical manifold 234 comprises an "E" configuration. The "E" configuration comprises a central rigid tube 366 with three stubs 368, 371, and 373 connected to tubes 232, 218 and 236, respectively. Diametrically across from the three stubs, a fourth stub 375 connects to tube 246 and thence to the auxiliary bag 244. The fourth stub 375 is asymmetrically placed along the tube 366. Because of the asymmetrical shape of the manifold, the manifold can be mounted in a shaped recess on the central core in only one direction. Each of the tubes 218, 232, 236 and 246 of the bag set 210 will consequently be reliably mounted at the proper valve 328, 330, 332 or a sensor (not shown).

The separation apparatus also comprises a controller 357 including a control unit (e.g. a microprocessor) and a memory unit for providing the microprocessor with information and programmed instructions relative to various separation protocols (e.g. a protocol for the separation of a plasma component and a blood cell component, or a protocol for the separation of a plasma component, a platelet component, and a red blood cell component) and to the operation of the apparatus in accordance with such separation protocols. In particular, the microprocessor is programmed for receiving information relative to the centrifugation speed(s) at which the rotor is to be rotated during the various stages of a separation process (e.g. stage of component separation, stage of a plasma component expression, stage of suspension of platelets in a plasma fraction, stage of a platelet component expression, etc), and information relative to the various transfer flow rates at which separated components are to be transferred from the separation bag 212 into the component bags 214, 216. The information relative to the various transfer flow rates can be expressed, for example, as hydraulic liquid flow rates in the hydraulic circuit, or as rotation speeds of the brushless DC motor 316 of the hydraulic pumping station 306. The microprocessor is further programmed for receiving, directly or through the memory, information from the pressure gauge 324 and from four pairs of photocell sensors (not shown) and for controlling the centrifuge motor 280, the brushless DC motor 316 of the pumping station 306, and the four sets of pinch valves 328, 330, 332 so as to cause the separation apparatus to operate along a selected separation protocol.

A first balancing means initially balances the rotor when the weights of the four separation bags 212 contained in the separation cells 278 are different. The first balancing means substantially comprises the same structural elements as the elements of the component transferring means described above, namely: four expandable hydraulic chambers 300 interconnected by a peripheral circular manifold 302, and a hydraulic liquid pumping station 306 for pumping hydraulic liquid into the hydraulic chambers 300 through a rotor duct 310, which is connected to the circular manifold 302. Under centrifugation forces, the hydraulic liquid will distribute unevenly in the four separation cells 278 depending on the difference in weight of the separation bags 212, and balance the rotor.

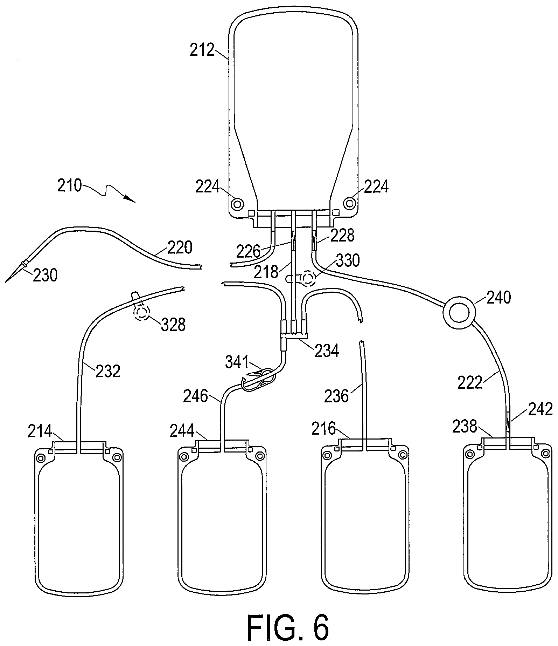

After the products are separated into the component bags 214, 216 (see FIG. 6) and waste product bag 244, the centrifuge is stopped and the bag set 210 is removed from the centrifuge. The tubes 232 and 236 are sealed, preferably by the application of radio frequency energy through the valves 330, 332, and removed from the bag set 210. A clamp 341 is used to close the tube 246 to the waste product bag 244. The separation bag 212, which now contains red blood cells, is suspended and the red blood cells drain by gravitational action through the filter 240. Leukocyte reduced red blood cells are collected in the red blood cell collection bag 238. When substantially all of the red blood cells have emptied out of the separation bag 212, the clamp 341 is removed from line 246, and the waste product bag is suspended above the separation bag, thereby allowing the waste product, e.g., white blood cells to drain into the filter 240, thereby pushing a residual amount of red blood cells out of the filter for collection. Because the volume of the waste product (white blood cells) is small, and because the filter is generally chosen to obstruct the flow of particles of the size of white blood cells, the waste product does not pass completely through the filter. Therefore, a greater volume of purified red blood cells is collected by using the white blood cell waste product to purge the filter of red blood cells.

The foregoing description of the present invention has been presented for purposes of illustration and description. Furthermore, the description is not intended to limit the invention to the form disclosed herein. Consequently, variations and modifications commensurate with the above teachings, and skill and knowledge of the relevant art, are within the scope of the present invention. The embodiments described hereinabove are further intended to explain best modes known of practicing the invention and to enable others skilled in the art to utilize the invention in such or other embodiments and with various modifications required by the particular application(s) or use(s) of the present invention. It is intended that the appended claims be construed to include alternative embodiments to the extent permitted by the prior art.

## Claims

1. A method for the leukoreduction of red blood cells comprising:
providing separated red blood cells recently removed from a donor;
pushing said separated red blood cells through a leukoreduction filter (120, 240);
collecting the red blood cells in a collection container (62, 238) after said step of pushing said high hematocrit red blood cells through said leukoreduction filter (120, 240); and **characterised by**:
diverting a predetermined quantity of another blood component into said leukoreduction filter (120, 240) after said collecting step to flush red blood cells from said filter (120, 240) into said collection container (62, 238).

2. The method of claim 1 further comprising
providing a blood component path (98) for another blood component other than red blood cells, said blood component path fluidly coupled to an outlet (50) of a blood processing vessel (12),
selectively fluidly coupling said blood component path to a storage location (58) or to a red blood cell path (60), said blood component path being coupled to said red blood cell path between a red blood cell outlet (46) of said blood processing vessel and said filter, and
flowing said predetermined quantity of another said blood component through said blood component path into said filter.

3. A disposable bag and tubing set (8) for collecting blood components comprising
a blood processing vessel adapted to be mounted on a rotor (20) of a centrifugal blood separation apparatus (6),
at least one red blood cell collection bag (62),
a red blood cell path (46,60), comprising tubing, said red blood cell path coupling an outlet of said blood processing vessel to said red blood cell collection bag,
a filter (120) in said red blood cell path, said filter being interposed between said outlet of said blood cell processing vessel and said red blood cell collection bag,
a blood component path (98) for a blood component other than red blood cells, said blood component path fluidly coupled to a second outlet of said blood processing vessel and selectively fluidly coupled to a storage location (58) or to said red blood cell path, said blood component path being coupled to said red blood cell path between said outlet and said filter.

4. The disposable bag and tubing set of claim 3 wherein said storage location is fluidly connected to means (8) for returning fluid to a donor.

5. The disposable bag and tubing set of claim 3 wherein said storage location comprises a component collection bag.

6. An apparatus for separating blood, said apparatus comprising
a centrifuge rotor (20),
a blood processing vessel (12) adapted to be mounted on said rotor,
at least one red blood cell collection bag (62),
a red blood cell path (46,60), comprising tubing, said red blood cell path coupling an outlet of said blood processing vessel to said red blood cell collection bag,
a filter (120) in said red blood cell path, said filter being interposed between said outlet of said blood cell processing vessel and said red blood cell collection bag,
a blood component path (98) for a blood component other than red blood cells, said blood component path fluidly coupled to a second outlet of said blood processing vessel and selectively fluidly coupled to a storage location (58) or to said red blood cell path, said blood component path being coupled to said red blood cell path between said outlet and said filter.

7. The apparatus of claim 6 wherein said storage location is fluidly connected to means (8) for returning fluid to a donor.

8. The apparatus of claim 7 wherein said storage location comprises a component collection bag.

9. A method for collecting red blood cell comprising
centrifugally separating a quantity of whole blood into red blood cells and at least one other component,
at least temporarily collecting said other component in a collection bag (244),
passing said red blood cells through a filter (240) during said separating step,
collecting filtered red blood cells in a red blood cell collection bag (238),
passing said other component into said filter to displace red blood cells out of said filter into said collection bag.

10. The method of claim 9 further comprising
collecting said red blood cells, said other component and at least one additional blood component in a fluidly interconnected set of collection bags (214, 216, 238), and
removing a bag (214) containing said additional component from said set of collection bags before passing said other component into said filter.

11. The method of claim 10 further comprising hanging said other component collection bag (244) above said filter and said red cell collection bag (238), thereby pushing said red blood cells out of said filter.

12. The method of any one of claims 1, 2 or 9 to 11, the disposable bag and tubing set of any one of claims 3 to 6, or the apparatus of any one of claims 6 to 8, wherein said blood component other than red blood cells comprises buffy coat.

## Patentansprüche

1. Verfahren zur Leukoreduktion von Erythrozyten, umfassend:
Bereitstellen abgetrennter Erythrozyten, die einem Spender vor Kurzem entnommen wurden;
Drücken der abgetrennten Erythrozyten durch einen Leukoreduktionsfilter (120, 240);
Sammeln der Erythrozyten in einem Sammelbehälter (62, 238) nach dem Schritt des Drückens der Erythrozyten mit hohem Hämatokrit durch den Leukoreduktionsfilter (120, 240);
und durch Folgendes gekennzeichnet:
Abzweigen einer vorbestimmten Menge eines anderen Blutbestandteils in den Leukoreduktionsfilter (120, 240) nach dem Sammelschritt, um Erythrozyten vom Filter (120, 240) in den Sammelbehälter (62, 238) zu spülen.

2. Verfahren nach Anspruch 1, ferner umfassend
Bereitstellen eines Blutbestandteilpfads (98) für einen anderen Blutbestandteil als Erythrozyten, wobei der Blutbestandteilpfad fluidisch an einen Auslass (50) eines Blutverarbeitungsgefäßes (12) gekoppelt ist,
selektives fluidisches Koppeln des Blutbestandteilpfads an eine Speicherstelle (58) oder an einen Erythrozytenpfad (60), wobei der Blutbestandteilpfad an den Erythrozytenpfad zwischen einem Erythrozytenauslass (46) des Blutverarbeitungsgefäßes und dem Filter gekoppelt ist, und
Fließen der vorbestimmten Menge des anderen Blutbestandteils durch den Blutbestandteilpfad in den Filter.

3. Einwegbeutel- und Schlauchset (8) zum Sammeln von Blutbestandteilen, umfassend:
ein Blutverarbeitungsgefäß, das angepasst ist, um auf einen Rotor (20) einer Zentrifugalbluttrennungsvorrichtung (6) montiert zu werden,
mindestens einen Erythrozytensammelbeutel (62),
einen Erythrozytenpfad (46, 60), umfassend Schläuche, wobei der Erythrozytenpfad einen Auslass des Blutverarbeitungsgefäßes an den Erythrozytensammelbeutel koppelt,
einen Filter (120) im Erythrozytenpfad, wobei sich der Filter zwischen dem Auslass des Blutverarbeitungsgefäßes und dem Erythrozytensammelbeutel befindet,
einen Blutbestandteilpfad (98) für einen anderen Blutbestandteil als Erythrozyten, wobei der Blutbestandteilpfad fluidisch an einen zweiten Auslass des Blutverarbeitungsgefäßes und selektiv fluidisch an eine Speicherstelle (58) oder an den Erythrozytenpfad gekoppelt ist, wobei der Blutbestandteilpfad zwischen dem Auslass und dem Filter an den Erythrozytenpfad gekoppelt ist.

4. Einwegbeutel- und Schlauchset nach Anspruch 3, wobei die Speicherstelle fluidisch mit Mittel (8) zum Zurückführen von Fluid zu einem Spender verbunden ist.

5. Einwegbeutel- und Schlauchset nach Anspruch 3, wobei die Speicherstelle einen Bestandteilsammelbeutel umfasst.

6. Vorrichtung zur Bluttrennung, wobei die Vorrichtung Folgendes umfasst:
einen Zentrifugenrotor (20),
ein Blutverarbeitungsgefäß (12), das angepasst ist, um auf dem Rotor montiert zu werden,
mindestens einen Erythrozytensammelbeutel (62),
einen Erythrozytenpfad (46, 60), umfassend Schläuche, wobei der Erythrozytenpfad einen Auslass des Blutverarbeitungsgefäßes an den Erythrozytensammelbeutel koppelt,
einen Filter (120) im Erythrozytenpfad, wobei sich der Filter zwischen dem Auslass des Blutverarbeitungsgefäßes und dem Erythrozytensammelbeutel befindet,
einen Blutbestandteilpfad (98) für einen anderen Blutbestandteil als Erythrozyten, wobei der Blutbestandteilpfad fluidisch an einen zweiten Auslass des Blutverarbeitungsgefäßes und selektiv fluidisch an eine Speicherstelle (58) oder an den Erythrozytenpfad gekoppelt ist, wobei der Blutbestandteilpfad zwischen dem Auslass und dem Filter an den Erythrozytenpfad gekoppelt ist.

7. Vorrichtung nach Anspruch 6, wobei die Speicherstelle fluidisch an Mittel (8) zum Zurückführen von Fluid zu einem Spender verbunden ist.

8. Vorrichtung nach Anspruch 7, wobei die Speicherstelle einen Bestandteilsammelbeutel umfasst.

9. Verfahren zum Sammeln von Erythrozyten, umfassend:
zentrifugales Abtrennen einer Menge von Vollblut in Erythrozyten und mindestens eines anderen Bestandteils,
wenigstens zeitweises Sammeln des anderen Bestandteils in einem Sammelbeutel (244),
Leiten der Erythrozyten durch einen Filter (240) während des Abtrennschritts,
Sammeln gefilterter Erythrozyten in einem Erythrozytensammelbeutel (238),
Leiten des anderen Bestandteils in den Filter, um Erythrozyten aus dem Filter in den Sammelbeutel zu verlagern.

10. Verfahren nach Anspruch 9, ferner umfassend:
Sammeln der Erythrozyten, des anderen Bestandteils und mindestens eines zusätzlichen Blutbestandteils in einer fluidisch miteinander verbundenen Reihe von Sammelbeuteln (214, 216, 238), und
Entfernen eines Beutels (214), der den zusätzlichen Bestandteil der Reihe der Sammelbeutel enthält, bevor der andere Bestandteil in den Filter geleitet wird.

11. Verfahren nach Anspruch 10, ferner umfassend ein Aufhängen des anderen Bestandteilsammelbeutels (244) über den Filter und den Erythrozytensammelbeutel (238), wodurch die Erythrozyten aus dem Filter gedrückt werden.

12. Verfahren nach jedem beliebigen der Ansprüche 1, 2 oder 9 bis 11, das Einwegbeutel- und Schlauchset nach einem der Ansprüche 3 bis 6, oder die Vorrichtung nach einem der Ansprüche 6 bis 8, wobei der andere Blutbestandteil als Erythrozyten einen Buffy-Coat umfasst.

## Revendications

1. Procédé de leucoréduction de globules rouges comprenant les étapes consistant à :
disposer de globules rouges séparés récemment prélevés sur un donneur,
faire passer lesdits globules rouges par un filtre de leucoréduction (120, 240),
recueillir les globules rouges dans un contenant de recueil (62, 238) après ladite étape consistant à faire passer lesdits globules rouges ayant un taux d'hématocrite élevé par ledit filtre de leucoréduction (120, 240) ; et **caractérisé par** l'étape consistant à :
dévier une quantité prédéterminée d'un autre composant sanguin pour la faire passer dans ledit filtre de leucoréduction (120, 240) après ladite étape de recueil afin d'entraîner les globules rouges issus dudit filtre (120, 240) dans ledit contenant de recueil (62, 238).

2. Procédé selon la revendication 1 comprenant en outre les étapes consistant à :
assurer une voie de passage de composant sanguin (98) pour un autre composant sanguin, différent des globules rouges, ladite voie de passage de composant sanguin étant en communication fluidique avec un orifice de sortie (50) d'un récipient de traitement du sang (12),
mettre en communication fluidique, de manière sélective, ladite voie de passage de composant sanguin avec un emplacement de stockage (58) ou avec une voie de passage de globules rouges (60), ladite voie de passage de composant sanguin étant en communication avec ladite voie de passage de globules rouges entre un orifice de sortie de globules rouges (46) dudit récipient de traitement du sang et ledit filtre, et
faire circuler ladite quantité prédéterminée dudit autre composant sanguin dans ladite voie de passage de composant sanguin pour l'apporter audit filtre.

3. Ensemble de poche(s) et tubes jetables (8) destiné au recueil de composants sanguins, comprenant :
un récipient de traitement du sang conçu pour être monté sur un rotor (20) d'un appareil de séparation de sang par centrifugation (6),
au moins une poche de recueil de globules rouges (62),
une voie de passage de globules rouges (46, 60), comprenant des tubes, ladite voie de passage de globules rouges mettant en communication un orifice de sortie dudit récipient de traitement de sang avec ladite poche de recueil de globules rouges,
un filtre (120) présent dans ladite voie de passage de globules rouges, ledit filtre étant interposé entre ledit orifice de sortie dudit récipient de traitement du sang et ladite poche de recueil de globules rouges,
une voie de passage de composant sanguin (98) pour un composant sanguin autre que les globules rouges, ladite voie de passage de composant sanguin étant en communication fluidique avec un deuxième orifice de sortie dudit récipient de traitement du sang et en communication fluidique de manière sélective avec un emplacement de stockage (58) ou ladite voie de passage de globules rouges, ladite voie de passage de composant sanguin étant en communication avec ladite voie de passage de globules rouges entre ledit orifice de sortie et ledit filtre.

4. Ensemble de poche(s) et tubes jetables selon la revendication 3, dans lequel ledit emplacement de stockage est en communication fluidique avec des moyens (8) permettant de restituer du fluide à un donneur.

5. Ensemble de poche(s) et tubes jetables selon la revendication 3, dans lequel ledit emplacement de stockage comprend une poche de recueil de composant.

6. Appareil destiné à séparer le sang, ledit appareil comprenant :
un rotor centrifuge (20),
un récipient de traitement du sang (12) conçu pour être monté sur ledit rotor,
au moins une poche de recueil de globules rouges (62),
une voie de passage de globules rouges (46, 60), comprenant des tubes, ladite voie de passage de globules rouges mettant en communication un orifice de sortie dudit récipient de traitement du sang avec ladite poche de recueil de globules rouges,
un filtre (120) présent dans ladite voie de passage de globules rouges, ledit filtre étant interposé entre ledit orifice de sortie dudit récipient de traitement du sang et ladite poche de recueil de globules rouges,
une voie de passage de composant sanguin (98) pour un composant sanguin autre que les globules rouges, ladite voie de passage de composant sanguin étant en communication fluidique avec un deuxième orifice de sortie dudit récipient de traitement du sang et en communication fluidique de manière sélective avec un emplacement de stockage (58) ou ladite voie de passage de globules rouges, ladite voie de passage de composant sanguin étant en communication avec ladite voie de passage de globules rouges entre ledit orifice de sortie et ledit filtre.

7. Appareil selon la revendication 6, dans lequel ledit emplacement de stockage est en communication fluidique avec des moyens (8) permettant de restituer du fluide à un donneur.

8. Appareil selon la revendication 7, dans lequel l'emplacement de stockage comprend une poche de recueil de composant.

9. Procédé de recueil de globules rouges comprenant :
la séparation centrifuge d'une quantité de sang complet en globules rouges et au moins un autre composant,
le recueil, au moins temporairement, dudit autre composant dans une poche de recueil (244),
le passage desdits globules rouges par un filtre (240) pendant ladite étape de séparation,
le recueil de globules rouges filtrés dans une poche de recueil de globules rouges (238),
le passage dudit autre composant dans ledit filtre pour déplacer les globules rouges hors dudit filtre vers l'intérieur de ladite poche de recueil.

10. Procédé selon la revendication 9 comprenant en outre :
le recueil desdits globules rouges, dudit autre composant et d'au moins un composant sanguin supplémentaire dans un ensemble de poches de recueil en communication fluidique les unes avec les autres (214, 216, 238), et
le retrait d'une poche (214) contenant ledit composant supplémentaire hors dudit ensemble de poches de recueil avant de faire passer ledit autre composant dans ledit filtre.

11. Procédé selon la revendication 10, comprenant en outre l'accrochage de ladite poche de recueil de l'autre composant (244) au-dessus dudit filtre et de ladite poche de recueil de globules rouges (238), faisant ainsi passer lesdits globules rouges hors dudit filtre.

12. Procédé selon l'une quelconque des revendications 1, 2 ou 9 à 11, ensemble de poche(s) et tubes jetables selon l'une quelconque des revendications 3 à 6 ou appareil selon l'une quelconque des revendications 6 à 8, dans lesquels ledit composant sanguin autre que des globules rouges comprend une couche leuco-plaquettaire.
